# EUROPÄISCHE PATENTSCHRIFT

(11) **EP 2 465 460 B1**
(45) Veröffentlichungstag und Bekanntmachung des Hinweises auf die Patenterteilung: **26.03.2014**
(21) Anmeldenummer: 10195068.1
(22) Anmeldetag: 15.12.2010
(51) Int. Cl.: A61B 19/00, A61C 19/00, A61B 19/02

(54) **Reinigungs- oder Pflegekassette für medizinische, insbesondere zahnärztliche, Instrumente**
Cleaning or maintaining cassette for medical, in particular dental instruments
Cassette de nettoyage ou d'entretien pour instruments médicaux, notamment dentaires

(43) Veröffentlichungstag der Anmeldung: 20.06.2012
(73) Patentinhaber: W & H Dentalwerk Bürmoos GmbH, 5111 Bürmoos (AT)
(72) Erfinder: Meburger, Jürgen, 5112, Lamprechtshausen (AT)

(56) Entgegenhaltungen:
- EP-A1- 1 779 800
- WO-A1-2007/066086
- DE-A1- 19 913 943
- US-A- 4 990 087

## Beschreibung

Die vorliegende Erfindung bezieht sich auf eine Reinigungs- oder Pflegekassette für medizinische, insbesondere zahnärztliche, Instrumente nach dem Oberbegriff des Anspruchs 1.

Derartige Reinigungs- oder Pflegekassetten dienen zur Aufnahme von zu reinigenden und/ oder pflegenden medizinischen, insbesondere zahnärztlichen, Instrumenten in Reinigungs- oder Pflegegeräten.

Als zu reinigende Instrumente werden insbesondere gerade, gebogene, oder pistolenförmige Handstücke als auch Teile von Handstücken, z. B. Handstückköpfe mit einer Werkzeugaufnahme zur Aufnahme eines Behandlungswerkzeugs, Adapter und Kupplungen verstanden. Durch die Handstücke erstrecken sich oftmals Versorgungsleitungen zum Antrieb eines Behandlungswerkzeugs sowie Fluidleitungen. Bei den Leitungen handelt es sich insbesondere um Getriebekanäle oder Fluidkanäle für Luft, Wasser oder Spray.

Nach dem Betrieb der oben angeführten Instrumente bedarf es in regelmäßigen Abständen einer Reinigung und Pflege der Leitungen, insbesondere der Antriebskanäle sowie der Fluidkanäle. Hierzu stehen dem Anwender eine Vielzahl an Reinigung- und Pflegegeräte zu Verfügung.

Unter Reinigungs- oder Pflegegerät sind Vorrichtungen zu verstehen, welche die medizinischen, insbesondere zahnärztlichen, Instrumente reinigen, insbesondere von Verschmutzungen befreien, desinfizieren, sterilisieren und/ oder pflegen. Zur Reinigung und/ oder Pflege der medizinischen, insbesondere zahnärztlichen, Instrumente können Fluide, insbesondere Flüssigkeiten, wie zum Beispiel Kaltwasser oder Heißwasser, oder Gase, wie zum Beispiel Wasserdampf oder Druckluft, Desinfektionsmittel, Sterilisationsmittel, oder Schmiermittel, wie zum Beispiel natürliche oder synthetische Schmieröle, verwendet werden. Diese Geräte weisen, neben einer Vorrichtung zur Außenreinigung, insbesondere eine Schnittstelle zur Reinigung der im Inneren des Handstücks angeordneten Versorgungs- und Fluidleitungen auf.

Die medizinischen, insbesondere zahnärztlichen, Instrumente werden vorzugsweise mittels einer Reinigungs- oder Pflegekassette in der Reinigungs- oder Pflegekammer des Reinigungs- oder Pflegegeräts aufgenommen und insbesondere zu der Schnittstelle des Reinigungs- oder Pflegegeräts zur Reinigung und Pflege der Versorgungs- und Fluidleitungen der medizinischen, insbesondere zahnärztlichen, Instrumente positioniert. Eine derartige Reinigungs- oder Pflegekassette ist aus der EP 1779800 B1 bekannt.

Diese bekannte Reinigungs- oder Pflegekassette weist ein Gehäuse mit einem oberen Gehäuseteil, insbesondere einem Deckel, und einem zweiten Gehäuseteil, insbesondere einem Basisteil, auf. Zwischen beiden Gehäuseteilen ist eine Anschlussvorrichtung zur Aufnahme zumindest eines medizinischen, insbesondere zahnärztlichen, Instruments angeordnet. Zum Verschließen und Öffnen der Reinigungskammer ist der Deckel am oberen Rand des Basisteils um eine Drehachse schwenkbar gelagert. Da das Verbinden des medizinischen, insbesondere zahnärztlichen, Instruments mit der Anschlussvorrichtung in einer etwa vertikalen Pflegestellung für den Anwender umständlich ist, ist die Anschlussvorrichtung um eine horizontale Querachse zwischen einer Reinigungsstellung in eine in etwa 45 Grad zur vertikalen Achse der Reinigungs-oder Pflegekassette angeordneten Bedienungsstellung am unteren Gehäuseteil schwenkbar gelagert. Eine Mitnehmervorrichtung dient zur Übertragung der Drehbewegung des Deckels auf die Anschlussvorrichtung. Dreht der Anwender den Deckel, so dreht die Mitnehmervorrichtung die Anschlussvorrichtung um eine Drehachse, so dass die Anschlussvorrichtung eine Bedienungsstellung einnimmt. Dies erleichtert dem Anwender das Aufstecken oder Lösen der medizinischen Instrumente.

Als nachteilig dieser Ausgestaltung der Reinigungs- oder Pflegekassette erweist sich die Bedienung, insbesondere das Beladen, der Reinigungskammer mit den medizinischen, insbesondere zahnärztlichen Instrumenten. Um die Kassette, insbesondere die Anschlussvorrichtung der Reinigungs- oder Pflegekassette, für den Anwender in gewünschter Weise, insbesondere in der Bedienungsstellung, zugänglich zu machen, muss der Anwender die Reinigungs- oder Pflegekassette richtig positionieren, das heißt er muss darauf achten, dass beim Öffnen der Kassette die Oberseite, nämlich der Deckel, oberhalb des Basisteils angeordnet ist.

Einen weiteren Nachteil der im Stand der Technik bekannten Ausführungsform stellt die Abhängigkeit der Schwenkfunktion von einem Gehäuseteil, nämlich dem Deckel, dar. Um die Anschlussvorrichtung der Reinigungs- oder Pflegekassette von der Pflegestellung in die Bedienungsstellung zu schwenken, muss der Anwender den Deckel gegenüber dem Basisteil verdrehen, so dass die an den Deckel gekoppelte Mitnehmervorrichtung die Anschlussvorrichtung um eine Drehachse in die Bedienungsstellung schwenkt. Verdreht der Anwender hingegen das Basisteil gegenüber dem Deckel erfolgt keine Übertragung der Drehbewegung auf die Anschlussvorrichtung. Die Anschlussvorrichtung verbleibt somit in der Pflegestellung.

Ein Reinigungs- oder Pflegegerät mit einem schwenkbar gelagerten Träger für medizinische Instrumente ist aus der DE 199 13 943 A1 bekannt, die die Merkmale des Oberbegriffs von Anspruch 1 offenbart.

Dieses bekannte Reinigungs- oder Pflegegerät weist ein Gehäuse auf, in welchem ein Träger zur Aufnahme mehrerer medizinischer Instrumente frei drehbar gelagert ist. Hierzu ist der als Welle ausgebildete Träger an den Seitenflächen des Innengehäuses in Schwenklagern gelagert. Um den Träger von einer Reinigungs- oder Pflegestellung in eine Bedienungsstellung zu schwenken, weist das Gerät einen Schwenkantrieb auf, welcher über eine Tür eines Reinigungs- oder Pflegeraums aktivierbar ist. Öffnet der Anwender die Tür zu dem Reinigungs- oder Pflegeraum, so dreht der Schwenkantrieb den Träger um seine Drehachse in eine Bedienungsstellung.

Auch bei diesem ausgebildeten Schwenkantrieb ist der Träger für die medizinischen Instrumente lediglich durch ein Gehäuseteil, nämlich durch die Tür, betätigbar.

Der vorliegenden Erfindung liegt daher die Aufgabe zugrunde eine Reinigungs- oder Pflegekassette zu schaffen, welche es ermöglicht, die medizinischen, insbesondere zahnärztliche, Instrumente bedienungsfreundlich in der Reinigungs- oder Pflegekassette aufzunehmen. Des Weiteren soll eine einfache Herstellung der Reinigungs- oder Pflegekassette gewährleistet werden.

Die erfindungsgemäße Reinigungs- oder Pflegekassette ist in den Ansprüchen definiert.

Gemäß einem Ausführungsbeispiel weist die Reinigungs- oder Pflegekassette zur Aufnahme von medizinischen, insbesondere zahnärztlichen, Instrumenten ein Gehäuse mit zumindest einem ersten und zweiten Gehäuseteil, welche relativ zueinander drehbar miteinander verbunden sind, und zumindest eine zwischen den zwei Gehäuseteilen angeordnete Anschlussvorrichtung für zumindest ein medizinisches, insbesondere zahnärztliches, Instrument auf, wobei die Anschlussvorrichtung zwischen einer Reinigungsstellung und einer Bedienungsstellung, zum Einbringen und Entnehmen des zumindest einen medizinischen, insbesondere zahnärztlichen, Instruments, schwenkbar gelagert ist, wobei die Anschlussvorrichtung bei wahlweiser Betätigung des ersten oder zweiten Gehäuseteils von der Reinigungs- oder Bedienungsstellung in die Bedienungs- oder Reinigungsstellung schwenkbar ist. Dafür ist ein die Anschlussvorrichtung verschwenkender Schwenkantrieb vorgesehen, welcher durch das erste und zweite Gehäuseteil betätigbar ist.

Unter dem Begriff Reinigungs- oder Pflegekassette sind insbesondere Vorrichtungen zu verstehen, welche zur Aufnahme von medizinischen, insbesondere zahnärztlichen, Instrumenten, in Reinigungs- oder Pflegegeräten zur Reinigung, Pflege, Desinfektion und/ oder Sterilisation dienen. Des Weiteren können derartige Kassetten verwendet werden, um die medizinischen Instrumente in gereinigtem oder sterilem Zustand aufzubewahren.

Gemäß einem Ausführungsbeispiel bildet die Anschlussvorrichtung für das zumindest eine medizinische, insbesondere zahnärztliche, Instrument die Drehachse der Reinigungs- oder Pflegekassette. Die Anschlussvorrichtung weist hierzu insbesondere einen Träger mit vorzugsweise zwei Lagerzapfen zur Lagerung zumindest eines der beiden Gehäuseteile auf, um welche das Gehäuseteil drehbar ist. An dem Träger ist zumindest ein Anschlussstück angeordnet, um zumindest ein medizinisches, insbesondere zahnärztliches, Instrument aufzunehmen. Des Weiteren ist es möglich das medizinische, insbesondere zahnärztliche, Instrument über einen Anschlusszapfen mit dem Anschlussstück zu verbinden.

Das Anschlussstück der Anschlussvorrichtung ist vorzugsweise relativ zu dem Träger drehbar gelagert, so dass das mit dem Anschlussstück verbundene medizinische, insbesondere zahnärztliche, Instrument relativ zu dem Träger und insbesondere zu der Reinigungs- oder Pflegekassette drehbar ist. Hierdurch ist es möglich die aufgenommen medizinischen Instrumente, insbesondere zahnärztliche Hand- und Winkelstücke, platzsparend in der Reinigungs- oder Pflegekassette aufzunehmen. Das Anschlussstück selbst erstreckt sich vorzugsweise bis an die Rückseite des Trägers der Anschlussvorrichtung oder ist zumindest von der Rückseite durch eine Öffnung in dem Träger zugänglich.

Des Weiteren bildet die Anschlussvorrichtung vorzugsweise ein weiteres Gehäuseteil der Reinigungs- oder Pflegekassette. Hierdurch ist es möglich eine Medienzuführung eines Reinigungs- oder Pflegegeräts mit der Reinigungs- oder Pflegekassette, insbesondere direkt mit der Anschlussvorrichtung, zu koppeln. Dafür weist das zumindest eine Anschlussstück der Anschlussvorrichtung einen ersten Kupplungsbereich für ein medizinisches Instrument sowie einen zweiten Kupplungsbereich zur Verbindung mit einer Medienzuführung eines Reinigungs- oder Pflegegeräts auf. Des Weiteren beinhaltet das zumindest eine Anschlussstück zumindest einen, vorzugsweise vier, Medienkanäle, um den Versorgungs- und Fluidleitungen der angekuppelten medizinischen Instrumente Reinigungs- oder Pflegemittel des Reinigungs- oder Pflegegeräts zuzuführen.

Die Anschlussvorrichtung selbst ist, insbesondere um eine Querachse, zwischen einer Reinigungs- oder Pflegestellung und einer Bedienungsstellung drehbar gelagert. Bevorzugt liegt hierbei die Querachse der Anschlussvorrichtung in der Drehachse der zumindest zwei Gehäuseteile der Reinigungs- oder Pflegekassette.

Gemäß einem Ausführungsbeispiel weist der Schwenkantrieb der Reinigungs- oder Pflegekassette zumindest zwei Mitnehmer auf, wobei je ein Mitnehmer an dem ersten und zweiten Gehäuseteil angeordnet ist, so dass bei wahlweiser Betätigung des ersten oder zweiten Gehäuseteils der erste oder zweite Mitnehmer die Anschlussvorrichtung von der Reinigungs- oder Bedienungsstellung in die Bedienungs- oder Reinigungsstellung schwenkt. Die zumindest zwei Mitnehmer sind vorzugsweise als Mitnehmernuten ausgebildet. Alternativ können die Mitnehmer auch in Form eines Mitnehmerzapfen ausgestaltet sein.

Das erste und zweite Gehäuseteil weist jeweils zumindest zwei Lagerstellen zur Aufnahme der Drehachse der Reinigungs- oder Pflegekassette auf, wobei in montiertem Zustand eine erste Lagerstelle des ersten Gehäuseteils zwischen den zwei Lagerstellen des zweiten Gehäuseteils und die zweite Lagerstelle des ersten Gehäuseteils außerhalb der zwei Lagerstellen des zweiten Gehäuseteils angeordnet ist. Die zumindest zwei Mitnehmer des Schwenkantriebs sind vorzugsweise an je einer der zumindest zwei Lagerstellen des ersten oder zweiten Gehäuseteils angebracht. Die insbesondere als Mitnehmernuten ausgebildeten Mitnehmer sind hierbei durch Rücksprünge in den Lagerstellen ausgebildet.

Die Anschlussvorrichtung weist des Weiteren zumindest ein mit den Mitnehmern des ersten und zweiten Gehäuseteils zusammenwirkendes Element auf. Dieses ist insbesondere bei Ausgestaltung des Mitnehmers als Nut in Form eines Zapfens ausgebildet. Hierbei greift der Zapfen in die Mitnehmernut. Verdreht der Anwender somit das erste Gehäuseteil gegenüber dem zweiten Gehäuseteil erfolgt mittels der Mittnehmernut eine Übertragung der Drehbewegung auf den Zapfen der Anschlussvorrichtung. Die Anschlussvorrichtung wird somit von der Reinigungs- oder Pflegestellung in die Bedienungsstellung oder umgekehrt verschwenkt.

Gemäß einem bevorzugten Ausführungsbeispiel ist das erste und zweite Gehäuseteil um einen ersten Drehwinkel verschwenkbar, wobei die zumindest zwei Mitnehmer des ersten und zweiten Gehäuseteile derart ausgebildet sind, dass die Anschlussvorrichtung um einen zweiten Drehwinkel verschwenkbar ist, wobei der zweite Drehwinkel kleiner als der erste Drehwinkel, insbesondere der Öffnungswinkel der Reinigungs- oder Pflegekassette, ist. Hierdurch wird die Anschlussvorrichtung in der Bedienungsstellung beabstandet von beiden Gehäuseteilen positioniert.

Des Weiteren weist der Schwenkantrieb vorzugsweise zumindest ein Rastelement auf, um das verschwenkte erste oder zweite Gehäuseteil und/ oder die verschwenkte Anschlussvorrichtung in der verschwenkten Position, insbesondere in der Bedienungsstellung, zu fixieren. Das zumindest eine Rastelement ist hierzu vorzugsweise durch einen Vorsprung an dem ersten und/ oder zweiten Gehäuseteil ausgebildet.

Gemäß einem weiteren bevorzugten Ausführungsbeispiel ist das Gehäuse, insbesondere das erste und zweite Gehäuseteil, der Reinigungs- oder Pflegekassette und die Anschlussvorrichtung, zumindest der Träger der Anschlussvorrichtung, im montierten Zustand drehsymmetrisch ausgebildet. Durch die Drehung des ersten Gehäuseteils und der Anschlussvorrichtung um einen Winkel von 180 Grad um die Drehsymmetrieachse der Reinigungs- oder Pflegekassette, wird das erste Gehäuseteil auf dem zweiten Gehäuseteil und die Anschlussvorrichtung auf sich selbst abgebildet. Auf Grund dieser Ausgestaltung beider Gehäuseteile der Kassette ist es möglich das erste und zweite Gehäuseteil durch ein identisches Bauteil auszugestalten.

Gemäß einem weiteren bevorzugten Ausführungsbeispiel ist des Weiteren der Schwenkantrieb der Reinigung- oder Pflegekassette drehsymmetrisch zu der Drehsymmetrieachse der Reinigungs- oder Pflegekassette ausgebildet. Durch die Drehung des Schwenkantriebs um einen Winkel von 180 Grad um die Drehsymmetrieachse der Reinigungs- oder Pflegekassette wird der Schwenkantrieb wieder auf sich selbst abgebildet.

Die vorliegende Erfindung zeichnet sich durch eine Reihe erheblicher Vorteile aus.

Auf Grund der durch das erste und zweite Gehäuseteil betätigbaren Anschlussvorrichtung ist es möglich, die Anschlussvorrichtung der Reinigungs- oder Pflegekassette durch Öffnen des ersten oder zweiten Gehäuseteils in gleicher Weise zugänglich zu machen. Bei wahlweiser Betätigung des ersten oder zweiten Gehäuseteils wird die Anschlussvorrichtung von der Reinigungs- oder Bedienungsstellung in die Bedienungs- oder Reinigungsstellung geschwenkt.

Des Weiteren wird durch die drehsymmetrische Ausbildung des Gehäuses, insbesondere des ersten und zweiten Gehäuseteils, der Anschlussvorrichtung sowie insbesondere des Schwenkantriebs der Reinigungs- oder Pflegekassette, eine einfache Bedienung, insbesondere Bestückung der Anschlussvorrichtung mit den medizinischen, insbesondere zahnärztlichen, Instrumenten, gewährleistet. Eine selektive Positionierung der Reinigungs- oder Pflegekassette ist somit nicht notwendig. Mittels Öffnen des ersten und/ oder zweiten Gehäuseteils ist die Anschlussvorrichtung für den Anwender in der Bedienungsstellung zugänglich.

Einen weiteren Vorteil der Erfindung stellt die kostengünstige Fertigung des Gehäuses und des Schwenkantriebs der Reinigungs- oder Pflegekassette dar. Durch die Ausgestaltung beider Gehäuseteile durch zwei Bauteile mit identischer Form, ist es möglich das zweiteilige Gehäuse mittels einem einzigen Fertigungswerkzeug, bzw. einer einzigen Fertigungsmaschine herzustellen.

Im Rahmen der Erfindung versteht es sich selbstverständlich, dass die Reinigungs- oder Pflegekassette nicht auf das oben beschriebene Ausführungsbeispiel beschränkt ist.

Nachfolgend wird die Erfindung anhand eines Ausführungsbeispiels und in Verbindung mit den beigefügten Zeichnungen erläutert.

Dabei zeigt:
Figur 1 ein Ausführungsbeispiel der Reinigungs- oder Pflegekassette in der Außenansicht,
Figur 2 die Reinigungs- oder Pflegekassette aus Figur 1 in der Bedienungsstellung,
Figur 3 ein Ausführungsbeispiel des ersten oder zweiten Gehäuseteils der Reinigungs-oder Pflegekassette,
Figur 4 ein Ausführungsbeispiel der Anschlussvorrichtung der Reinigungs- oder Pflegekassette;
die Figuren 5A bis 5C den Schwenkantrieb, das erste oder zweite Gehäuseteil und die Anschlussvorrichtung der Reinigungs- oder Pflegekassette zwischen der Reinigungs-oder Pflegestellung und der Bedienungsstellung in der Seitenansicht;

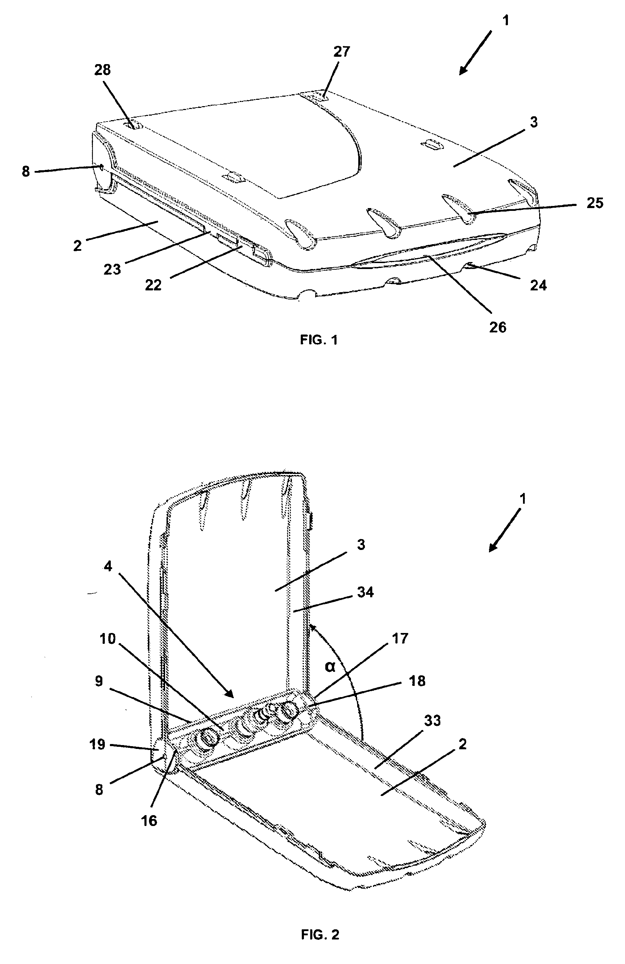

In Figur 1 ist ein Ausführungsbeispiel der Reinigungs- oder Pflegekassette 1 in der Außenansicht abgebildet. Hierbei wird insbesondere das Gehäuse mit einem ersten Gehäuseteil 2 sowie einem zweiten Gehäuseteil 3 gezeigt. Beide Gehäuseteile 2, 3 sind über eine Drehachse 8 drehbar miteinander verbunden. Sowohl das erste als auch das zweite Gehäuseteile 2, 3 weisen zumindest ein Verriegelungselement 22, 23 auf, so dass die zumindest zwei Gehäuseteile 2, 3 im geschlossenen Zustand miteinander lösbar verriegelbar sind. Des Weiteren weisen beide Gehäuseteil 2, 3 jeweils zumindest eine Öffnung 24, 25 zur Aufnahme zumindest einer Reinigungsdüse eines Reinigungs- oder Pflegegeräts in der Reinigungs- oder Pflegekassette 1 auf. Eine weitere Gehäuseöffnung 26, welche insbesondere durch Rücksprünge des ersten und zweiten Gehäuseteils 2, 3 gebildet wird, ermöglicht dem Anwender das Hintergreifen zumindest eines der beiden Gehäuseteile 2, 3, um die Reinigungs- oder Pflegekassette 1 zu öffnen. An den Außenseiten beider Gehäuseteile 2, 3 sind jeweils zumindest ein Gehäusevorsprung 27 sowie ein Gehäuserücksprung 28 angeordnet. Mittels des Gehäusevorsprungs 27 wird gewährleistet, dass bei Anordnung der Reinigungs- oder Pflegekassette 1 in einem Reinigungs- oder Pflegegerät ein Reinigungs- oder Pflegemittel an der Außenseite der Reinigungs- oder Pflegekassette 1 vorbeileitbar ist. Der Gehäusevorsprung 27 dient somit als Abstandshalter zwischen dem Gehäuse der Reinigungs- oder Pflegekassette 1 und der Innenwand des Reinigungs- oder Pflegegeräts. Der zumindest eine Gehäuserücksprung 28 an beiden Gehäuseteilen 2, 3 dient zum Stapeln mehrere Kassetten 1 übereinander. Hierbei greift ein Gehäusevorsprung 27 einer ersten Reinigungs- oder Pflegekassette 1 in einen Gehäuserücksprung 28 einer zweiten Reinigungs- oder Pflegekassette 1.

Figur 2 zeigt die Reinigungs- oder Pflegekassette 1, insbesondere die Anschlussvorrichtung 4 der Reinigungs- oder Pflegekassette 1, in der Bedienungsstellung 6. Die zwischen den zwei Gehäuseteilen 2, 3 angeordnete Anschlussvorrichtung 4 ist über zumindest zwei Lagerzapfen 29, 30 in den Lagerstellen 16, 17; 18, 19 des ersten und zweiten Gehäuseteils 2, 3 drehbar gelagert. Bevorzugt bildet die Anschlussvorrichtung 4 für das zumindest eine medizinische, insbesondere zahnärztliche, Instrument die Drehachse 8 der Reinigungs- oder Pflegekassette 1, um welche zumindest ein Gehäuseteil 2 relativ zu dem zweiten Gehäuseteil 3 um einen Drehwinkel, insbesondere um einen Öffnungswinkel α von 100 Grad, drehbar ist. Die Anschlussvorrichtung 4 selbst besteht insbesondere aus einem Träger 9 sowie zumindest aus einem Anschlussstück 10 für ein medizinisches, insbesondere zahnärztliches, Instrument. Das Anschlussstück 10 der Anschlussvorrichtung 4 ist relativ zu dem Träger 9 drehbar gelagert, so dass das mit dem Anschlussstück 10 verbundene medizinische, insbesondere zahnärztliche, Instrument relativ zu dem Träger 9 drehbar ist und platzsparend in der Reinigungs- oder Pflegekassette 1 aufgenommen werden kann.

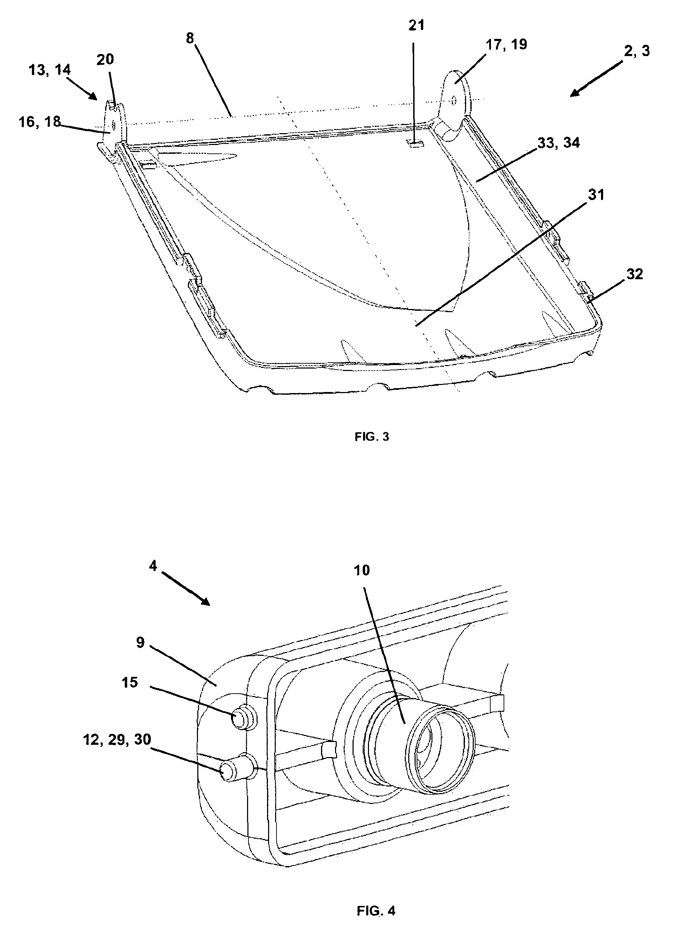

Das aus dem ersten und zweiten Gehäuseteil 2, 3 bestehende Gehäuse der Reinigungs-oder Pflegekassette 1 ist bevorzugt drehsymmetrisch ausgebildet. Durch die Drehung des ersten Gehäuseteils 2 um einen Winkel von 180 Grad um die Drehsymmetrieachse 31, siehe Figur 3, der Reinigungs- oder Pflegekassette 1, wird dieses auf dem zweiten Gehäuseteil 3 abgebildet. Durch diese Ausgestaltung beider Gehäuseteile 2, 3 der Kassette 1 ist es möglich, das erste und zweite Gehäuseteil 2, 3 durch eine identische Bauform auszubilden. In Figur 3 ist ein Ausführungsbeispiel des ersten oder zweiten Gehäuseteils 2, 3 der Reinigungs- oder Pflegekassette 1 gezeigt. Das erste und zweite Gehäuseteil 2, 3 weist jeweils zumindest zwei Lagerstellen 16, 17; 18, 19 zur Aufnahme der Drehachse 8 der Reinigungs- oder Pflegekassette 1 auf. Beide Lagerstellen 16, 17 oder 18, 19 des ersten oder zweiten Gehäuseteils 2, 3 sind hierzu unterschiedlich beabstandet zu der Drehsymmetrieachse 31 der Reinigungs- oder Pflegekassette 1, so dass in montiertem Zustand eine erste Lagerstelle 16 des ersten Gehäuseteils 2 zwischen den zwei Lagerstellen 18, 19 des zweiten Gehäuseteils 3 und die zweite Lagerstelle 17 des ersten Gehäuseteils 2 außerhalb der zwei Lagerstellen 18, 19 des zweiten Gehäuseteils 3 angeordnet ist. Des Weiteren weisen beide Gehäuseteile 2, 3 jeweils zumindest eine Kontaktfläche 32 auf, an der die zumindest eine Seitenfläche 33 des ersten Gehäuseteils 2 die zumindest eine Seitenfläche 34 des zweiten Gehäuseteils 3 kontaktiert.

In Figur 4 ist ein Ausführungsbeispiel der Anschlussvorrichtung 4 der Reinigungs- oder Pflegekassette 1 abgebildet. Die Anschlussvorrichtung 4 mit einem Träger 9 und zumindest einem Anschlussstück 10 ist drehbar um die Querachse 12 gelagert. In dem bevorzugten Ausführungsbeispiel ist die Anschlussvorrichtung 4, zumindest der Träger 9 der Anschlussvorrichtung 4, drehsymmetrisch zur Drehsymmetrieachse 31 der Reinigungs- oder Pflegekassette 1 ausgebildet. Hierdurch wird es ermöglicht, dass die mittels der Anschlussvorrichtung 4 in der Reinigungs- oder Pflegekassette 1 aufgenommen medizinischen Instrumente bei Öffnen des ersten und/ oder zweiten Gehäuseteils 2, 3 gleich zugänglich sind.

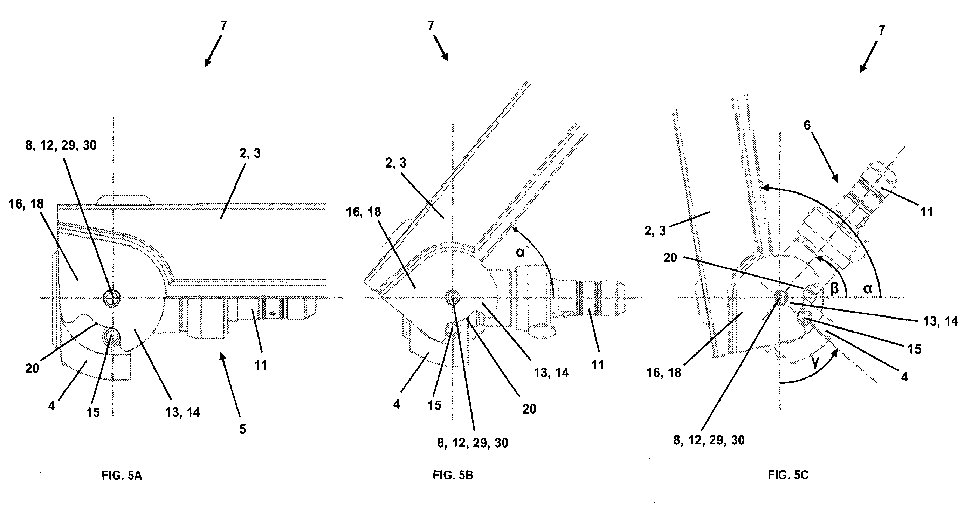

Die Figuren 5A bis 5C zeigen den Schwenkantrieb 7, das erste oder zweite Gehäuseteil 2, 3 und die Anschlussvorrichtung 4 der Reinigungs- oder Pflegekassette 1 zwischen der Reinigungs- oder Pflegestellung 5 und der Bedienungsstellung 6 in der Seitenansicht. Beide Gehäuseteile 2, 3 der Reinigungs- oder Pflegekassette 1 sind über eine Drehachse 8 drehbar miteinander verbunden. Die Anschlussvorrichtung 4 selbst ist drehbar um eine Querachse 12 gelagert. In diesem bevorzugten Ausführungsbeispiel liegt die Querachse 12 der Anschlussvorrichtung 4 in der Drehachse 8 der zumindest zwei Gehäuseteile 2, 3 der Reinigungs- oder Pflegekassette 1. Hierzu ist die Anschlussvorrichtung 4 über zwei Lagerzapfen 29, 30 in den zumindest zwei Lagerstellen 16, 18 des ersten und zweiten Gehäuseteils 2, 3 drehbar gelagert. Bevorzugt bildet die Anschlussvorrichtung 4 für das zumindest eine medizinische, insbesondere zahnärztliche, Instrument die Drehachse 8 der Reinigungs-oder Pflegekassette 1, insbesondere der beiden Gehäuseteile 2, 3.

Der Schwenkantrieb 7 der Reinigungs- oder Pflegekassette 1 weist zumindest zwei Mitnehmer 13, 14 auf, wobei je ein Mitnehmer 13, 14 an dem ersten und zweiten Gehäuseteil 2, 3 angeordnet ist, so dass bei wahlweiser Betätigung des ersten oder zweiten Gehäuseteils 2, 3 der erste oder zweite Mitnehmer 13, 14 die Anschlussvorrichtung 4 von der Reinigungs- oder Bedienungsstellung 5, 6 in die Bedienungs- oder Reinigungsstellung 6, 5 schwenkt. Die zumindest zwei Mitnehmer 13, 14 sind vorzugsweise als Mitnehmernuten 20, insbesondere in Form eines Rücksprungs, an den Lagerstellen 16, 18 ausgestaltet. Die Anschlussvorrichtung 4 weist des Weiteren zumindest ein mit den Mitnehmern 13, 14 des ersten und zweiten Gehäuseteils 2, 3 zusammenwirkendes Element 15 auf. Dieses ist insbesondere bei Ausgestaltung des Mitnehmers 13, 14 als Nut in Form eines Zapfens 15 ausgebildet. Hierbei greift der Zapfen 15 in die Mitnehmernut 20. Verdreht der Anwender somit das erste Gehäuseteil 2 gegenüber dem zweiten Gehäuseteil 3, erfolgt mittels der Mittnehmernut 20 eine Übertragung der Drehbewegung auf den Zapfen 15 der Anschlussvorrichtung 4. Die Anschlussvorrichtung 4 wird somit von der Reinigungs- oder Pflegestellung 5 in die Bedienungsstellung 6, oder umgekehrt, verschwenkt.

In Figur 5A ist die Anschlussvorrichtung 4 mit dem Anschlusszapfen 11 für das zumindest eine medizinische, insbesondere zahnärztliche, Instrument und das erste oder zweite Gehäuseteil 2, 3 der Reinigungs- oder Pflegekassette 1 in der Pflegestellung 5 gezeigt. Der mit der Mitnehmernut 20 des Schwenkantriebs 7 zusammenwirkende Zapfen 15 der Anschlussvorrichtung 4 ist in der Pflegestellung 5 vorzugsweise an einem ersten Ende der Mitnehmernut 20, welche sich vorzugsweise radial um die Drehachse 8 der Reinigungs- oder Pflegekassette 1 erstreckt, positioniert.

Durch das Schwenken des ersten oder zweiten Gehäuseteils 2, 3 um einen Drehwinkel α ', um die Reinigungs- oder Pflegekassette 1 zu öffnen, siehe insbesondere Figur 5B, wird die Mitnehmernut 20 des Schwenkantriebs 7 um den Winkel α' verdreht, wodurch der Zapfen 15 der Anschlussvorrichtung an einem zweiten Ende der Mitnehmernut 20 positioniert wird. Die Anschlussvorrichtung 4, insbesondere der Anschlusszapfen 11, verbleibt hierbei während und nach der Schwenkbewegung des ersten oder zweiten Gehäuseteils 2, 3 um den Winkel α' in der Pflegestellung 5. Die Reinigungs- oder Pflegekassette 1 kann somit geöffnet werden, ohne die Anschlussvorrichtung 4 mittels des Schwenkantriebs 7 zu betätigen.

Figur 5C zeigt die Anschlussvorrichtung 4 und das erste oder zweite Gehäuseteil 2, 3 der Reinigungs- oder Pflegekassette 1 in der Bedienungsstellung 6. Durch das Schwenken des ersten oder zweiten Gehäuseteils 2, 3 in den Öffnungswinkel α der Reinigungs- oder Pflegekassette 1, schwenkt die Mitnehmernut 20, insbesondere deren zweites Ende, den Zapfen 15 der Anschlussvorrichtung 4 um den Winkel y, wodurch die Anschlussvorrichtung 4 um den Drehwinkel β von der Reinigungs- oder Pflegestellung 5 in die Bedienungsstellung 6 geschwenkt wird.

Der Öffnungswinkel α der Reinigungs- oder Pflegekassette 1 beträgt vorzugsweise 100 Grad, wobei der Schwenkantrieb 7 derart ausgebildet ist, dass die Anschlussvorrichtung 4 um einen zweiten Drehwinkel β von vorzugsweise 50 Grad verschwenkbar ist.

Die Erfindung ist nicht auf das beschriebene Ausführungsbeispiel beschränkt, sondern umfasst alle Ausführungen, die das prinzipielle, sinngemäße Funktionsprinzip der Erfindung anwenden oder beinhalten. Insbesondere kann der Antrieb zum Schwenken der Anschlussvorrichtung zwischen der Reinigungs- oder Pflegestellung in die Bedienungsstellung durch andere bekannte Antriebe, wie z.B. Getriebe mit kämmenden Zahnrädern, ausgebildet sein. Des Weiteren ist es auch möglich, dass das erste und zweite Gehäuseteil die Anschlussvorrichtung unmittelbar beim Öffnen eines der beiden Gehäuseteile von der Reinigungs- oder Pflegestellung in die Bedienungsstellung schwenkt.

## Patentansprüche

1. Reinigungs- oder Pflegekassette (1) zur Aufnahme von medizinischen, insbesondere zahnärztlichen, Instrumenten, aufweisend ein Gehäuse mit zumindest einem ersten und zweiten Gehäuseteil (2, 3), welche relativ zueinander drehbar miteinander verbunden sind, und zumindest eine zwischen den zwei Gehäuseteilen (2, 3) angeordnete Anschlussvorrichtung (4) für zumindest ein medizinisches, insbesondere zahnärztliches, Instrument, wobei die Anschlussvorrichtung (4) zwischen einer Reinigungsstellung (5) und einer Bedienungsstellung (6) zum Einbringen und Entnehmen des zumindest einen medizinischen, insbesondere zahnärztlichen, Instruments, schwenkbar gelagert ist, wobei ein die Anschlussvorrichtung (4) verschwenkender Schwenkantrieb (7) vorgesehen ist, welcher durch wahlweise Betätigung des ersten oder zweiten Gehäuseteils (2, 3) betätigbar ist, wobei der Schwenkantrieb (7) zumindest zwei Mitnehmer (13, 14) aufweist, wobei je ein Mitnehmer (13, 14) an dem ersten und zweiten Gehäuseteil (2, 3) angeordnet ist, **dadurch gekennzeichnet, dass** bei Betätigung des ersten Gehäuseteils (2) nur der erste Mitnehmer (13)
und bei Betätigung des zweiten Gehäuseteils (3) nur der zweite Mitnehmer (14) die Anschtussvorrichtung (4) von der Reinigungs-in die Bedienungsstellung (6) oder von der Bedienungs- in die Reinigungsstellung (5) schwenkt.

2. Reinigungs- oder Pflegekassette (1) nach Anspruch 1, **dadurch gekennzeichnet, dass** die Anschlussvorrichtung (4) für das zumindest eine medizinische, insbesondere zahnärztliche, Instrument die Drehachse (8) für das zumindest eine erste und zweite Gehäuseteil (2, 3) der Reinigungs- oder Pflegekassette (1) bildet.

3. Reinigungs- oder Pflegekassette (1) nach Anspruch 1 oder 2, **dadurch gekennzeichnet, dass** die Anschlussvorrichtung (4) einen Träger (9) und zumindest ein Anschlussstück (10) aufweist, um zumindest ein medizinisches, insbesondere zahnärztliches Instrument, oder einen Anschlusszapfen (11) für ein medizinisches, insbesondere zahnärztliches, Instrument aufzunehmen.

4. Reinigungs- oder Pflegekassette (1) nach einem der vorherigen Ansprüche, **dadurch gekennzeichnet, dass** die Anschlussvorrichtung (4) drehbar um eine Querachse (12) gelagert ist, wobei bevorzugt die Querachse (12) der Anschlussvorrichtung (4) in der Drehachse (8) der zumindest zwei Gehäuseteile (2, 3) der Reinigungs- oder Pflegekassette (1) liegt.

5. Reinigungs- oder Pflegekassette (1) nach einem der vorherigen Ansprüche, **dadurch gekennzeichnet, dass** die zumindest zwei Mitnehmer (13, 14) je eine Mitnehmernut oder einen Mitnehmerzapfen aufweisen.

6. Reinigungs- oder Pflegekassette (1) nach einem der vorherigen Ansprüche, **dadurch gekennzeichnet, dass** die Anschlussvorrichtung (4) zumindest ein mit den Mitnehmern (13, 14) des ersten und zweiten Gehäuseteils (2, 3) zusammenwirkendes Element (15) aufweist.

7. Reinigungs- oder Pflegekassette (1) nach einem der vorherigen Ansprüche, **dadurch gekennzeichnet, dass** das erste und zweite Gehäuseteil (2, 3) um einen ersten Drehwinkel (α) verschwenkbar ist und dass die Mitnehmer (13, 14) derart ausgebildet sind, dass die Anschlussvorrichtung (4) um einen zweiten Drehwinkel (β) verschwenkbar ist, wobei der zweite Drehwinkel (β) kleiner als der erste Drehwinkel (α) ist.

8. Reinigungs- oder Pflegekassette (1) nach einem der vorherigen Ansprüche, **dadurch gekennzeichnet, dass** das erste und zweite Gehäuseteil (2, 3) jeweils zumindest zwei Lagerstellen (16, 17; 18, 19) zur Aufnahme der Drehachse (8) der Reinigungs- oder Pflegekassette (1) aufweist, wobei in montiertem Zustand eine erste Lagerstelle (16) des ersten Gehäuseteils (2) zwischen den zwei Lagerstellen (18, 19) des zweiten Gehäuseteils (3) und die zweite Lagerstelle (17) des ersten Gehäuseteils (2) außerhalb der zwei Lagerstellen (18, 19) des zweiten Gehäuseteils (3) angeordnet ist.

9. Reinigungs- oder Pflegekassette (1) nach Anspruch 8, **dadurch gekennzeichnet, dass** der erste oder zweite Mitnehmer (13, 14) des Schwenkantriebs (7) an je einer der zumindest zwei Lagerstellen (16, 17; 18,19) angebracht ist.

10. Reinigungs- oder Pflegekassette (1) nach Anspruch 9, **dadurch gekennzeichnet, dass** zumindest einer der Mitnehmer (13, 14) durch einen Rücksprung (20) in einer der Lagerstellen (16, 17; 18, 19) gebildet ist.

11. Reinigungs- oder Pflegekassette (1) nach einem der vorherigen Ansprüche, **dadurch gekennzeichnet, dass** der Schwenkantrieb (7) zumindest ein Rastelement (21) aufweist, um das verschwenkte erste oder zweite Gehäuseteil (2, 3) und/ oder die verschwenkte Anschlussvorrichtung (4) in der verschwenkten Position zu fixieren.

12. Reinigungs- oder Pflegekassette (1) nach Anspruch 11, **dadurch gekennzeichnet, dass** das Rastelement (21) durch zumindest einen Vorsprung an dem ersten und/ oder zweiten Gehäuseteil (2, 3) ausgebildet ist.

13. Reinigungs- oder Pflegekassette (1) nach einem der vorherigen Ansprüche, **dadurch gekennzeichnet, dass** das Gehäuse und die Anschlussvorrichtung (4) der Reinigungs- oder Pflegekassette (1) drehsymmetrisch ausgebildet sind.

14. Reinigungs- oder Pflegekassette (1) nach einem der vorherigen Ansprüche, **dadurch gekennzeichnet, dass** der Schwenkantrieb (7) der Reinigungs- oder Pflegekassette (1) drehsymmetrisch ausgebildet ist.

## Claims

1. A cleaning or care cassette (1) for the receptacle of medical, in particular dental, instruments, comprising a housing having at least one first and one second housing part (2, 3), which are connected so they can rotate in relation to one another, and at least one connecting device (4) arranged between the two housing parts (2, 3) for at least one medical, in particular dental, instrument, wherein the connecting device (4) is mounted so it can be pivoted between a cleaning position (5) and an operating position (6) for introducing and removing the at least one medical, in particular dental, instrument, wherein a pivot drive (7), which is capable of pivoting the connecting device (4) is provided, this pivot drive being operable by optional operation of the first or second housing part (2, 3), wherein the pivot drive (7) has at least two carrier elements (13, 14), wherein one carrier element (13, 14) each being arranged on the first and second housing parts (2, 3), **characterized in that** in operation of the first housing part (2), only the first carrier element (13) and in operation of the second housing part (3), only the second carrier element (14) pivots the connecting device (4) out of the cleaning position into the operating position (6) or out of the operating position into the cleaning position (5).

2. The cleaning or care cassette (1) according to claim 1, **characterized in that** the connecting device (4) for the at least one medical, in particular dental, instrument forms the axis of rotation (8) for the at least one first and second housing part (2, 3) of the cleaning or care cassette (1).

3. The cleaning or care cassette (1) according to claim 1 or 2, **characterized in that** the connecting device (4) has a carrier (9) and at least one connecting piece (10) to accommodate at least one medical, in particular dental, instrument or a connecting pin (11) for a medical, in particular dental, instrument.

4. The cleaning or care cassette (1) according to any one of the preceding claims, **characterized in that** the connecting device (4) is rotatable about a transverse axis (12), wherein preferably the transverse axis (12) of the connecting device (4) lies in the axis of rotation (8) of the at least two housing parts (2, 3) of the cleaning or care cassette (1).

5. The cleaning or care cassette (1) according to any one of the preceding claims, **characterized in that** each of the at least two carrier elements (13, 14) has one carrier groove or one carrier pin.

6. The cleaning or care cassette (1) according to any one of the preceding claims, **characterized in that** the connecting device (4) has at least one element, which cooperates with the carrier elements (13, 14) of the first and second housing parts (2, 3).

7. The cleaning or care cassette (1) according to any one of the preceding claims, **characterized in that** the first and second housing parts (2, 3) are pivotable about a first angle of rotation (α), and the carrier elements (13, 14) are designed so that the connecting device (4) is pivotable about a second angle of rotation (β), the second angle of rotation (β) being smaller than the first angle of rotation (α).

8. The cleaning or care cassette (1) according to any one of the preceding claims, **characterized in that** each of the first and second housing parts (2, 3) has at least two bearing points (16, 17; 18, 19) to accommodate the axis of rotation (8) of the cleaning or care cassette (1), wherein a first bearing point (16) of the first housing part (2) is arranged between the two bearing points (18, 19) of the second housing part (3) in the installed state, and the second bearing point (17) of the first housing part (2) is arranged outside of the two bearing points (18, 19) of the second housing part (3).

9. The cleaning or care cassette (1) according to claim 8, **characterized in that** the first or second carrier element (13, 14) of the pivot drive (7) is mounted on one of the at least two bearing points (16, 17; 18,19).

10. The cleaning or care cassette (1) according to claim 9, **characterized in that** at least one of the carrier elements (13, 14) is formed by a setback (20) in one of the bearing points (16, 17; 18, 19).

11. The cleaning or care cassette (1) according to any one of the preceding claims, **characterized in that** the pivot drive (7) has at least one latching element (21) to secure the pivoted first or second housing part (2, 3) and/or the pivoted connecting device (4) in the pivoted position.

12. The cleaning or care cassette (1) according to claim 11, **characterized in that** the latching element (21) is formed by at least one protrusion on the first and/or second housing parts (2, 3).

13. The cleaning or care cassette (1) according to any one of the preceding claims, **characterized in that** the housing and the connecting device (4) of the cleaning or care cassette (1) are designed to be rotationally symmetrical.

14. The cleaning or care cassette (1) according to any one of the preceding claims, **characterized in that** the pivot drive (7) of the cleaning or care cassette (1) is designed to be rotationally symmetrical.

## Revendications

1. Cassette de nettoyage ou d'entretien (1) destinée à recevoir des instruments médicaux, en particulier dentaires, présentant un boîtier comportant au moins une première et une seconde partie de boîtier (2, 3) qui sont raccordées entre elles avec possibilité de rotation l'une par rapport à l'autre et au moins un dispositif de raccordement (4) disposé entre les deux parties de boîtier (2, 3) pour au moins un instrument médical, en particulier dentaire, le dispositif de raccordement (4) s'appuyant en pivotement entre une position de nettoyage (5) et une position d'utilisation (6) pour introduire et sortir l'au moins un instrument médical, en particulier dentaire, étant prévue une commande pivotante (7) faisant pivoter le dispositif de raccordement (4) et qui est actionnable par actionnement au choix de la première ou de la seconde partie de boîtier (2, 3), la commande pivotante (7) présentant au moins deux entraîneurs (13, 14), chacun des entraîneurs (13, 14) étant disposé sur la première et la seconde partie du boîtier (2, 3), **caractérisée en ce que**, en cas d'actionnement de la première partie du boîtier (2), seul le premier entraîneur (13) et, en cas d'actionnement de la seconde partie du boîtier (3), seul le second entraîneur (14) fait pivoter le dispositif de raccordement (4) de la position de nettoyage à la position d'utilisation (6) ou de la position d'utilisation à la position de nettoyage (5).

2. Cassette de nettoyage ou d'entretien (1) selon la revendication 1, **caractérisée en ce que** le dispositif de raccordement (4) pour l'au moins un instrument médical, en particulier dentaire, constitue l'axe de rotation (8) pour l'au moins une première et seconde partie du boîtier (2, 3) du dispositif de nettoyage ou d'entretien (1).

3. Cassette de nettoyage ou d'entretien (1) selon la revendication 1 ou 2, **caractérisée en ce que** le dispositif de raccordement (4) présente un support (9) et au moins une pièce de raccordement (10) pour recevoir au moins un instrument médical, en particulier dentaire, ou un tourillon de raccordement (11) pour au moins un instrument médical, en particulier dentaire.

4. Cassette de nettoyage ou d'entretien (1) selon une des revendications précédentes, **caractérisée en ce que** le dispositif de raccordement (4) s'appuie en rotation autour d'un axe transversal (12), l'axe transversal (12) du dispositif de raccordement (4) se trouvant de préférence dans l'axe de rotation (8) des au moins deux parties de boîtier (2, 3) de la cassette de nettoyage ou d'entretien (1).

5. Cassette de nettoyage ou d'entretien (1) selon une des revendications précédentes, **caractérisée en ce que** les au moins deux entraîneurs (13, 14) présentent chacun une rainure d'entraînement ou un tourillon d'entraînement

6. Cassette de nettoyage ou d'entretien (1) selon une des revendications précédentes, **caractérisée en ce que** le dispositif de raccordement (4) présente au moins un élément (15) interagissant avec les entraîneurs (13, 14) de la première et de la seconde pièce du boîtier (2, 3).

7. Cassette de nettoyage ou d'entretien (1) selon une des revendications précédentes, **caractérisée en ce que** la première et la seconde pièce du boîtier (2, 3) peuvent pivoter à raison d'un premier angle de rotation (α) et que les entraîneurs (13, 14) sont conçus de manière à ce que le dispositif de raccordement (4) puisse pivoter à raison d'un second angle de rotation (β), le second angle de rotation (β) étant inférieur au premier angle de rotation (α).

8. Cassette de nettoyage ou d'entretien (1) selon une des revendications précédentes, **caractérisée en ce que** la première et la seconde partie de boîtier (2, 3) présentent respectivement au moins deux points d'appui (16, 17; 18, 19) destinés à recevoir l'axe de rotation (8) de la cassette de nettoyage et d'entretien (1 un premier point d'appui (16) de la première partie de boîtier (2) étant, en état monté, disposée entre les deux points d'appui (18, 19) de la seconde partie de boîtier (3) et le second point d'appui (17) de la première partie de boîtier (2) en dehors des deux points d'appui (18, 19) de la seconde partie de boîtier (3).

9. Cassette de nettoyage ou d'entretien (1) selon la revendication 8, **caractérisée en ce que** le premier ou le second entraîneur (13, 14) de la commande pivotante (7) est installé à chacun des au moins deux points d'appui (16, 17; 18,19).

10. Cassette de nettoyage ou d'entretien (1) selon la revendication 9, **caractérisée en ce qu'**au moins un des entraîneurs (13, 14) est constitué par un retrait (20) dans un des points d'appui (16, 17; 18, 19).

11. Cassette de nettoyage ou d'entretien (1) selon une des revendications précédentes, **caractérisée en ce que** la commande pivotante (7) présente au moins un élément d'enclenchement (21) pour fixer la première ou seconde partie de boîtier (2, 3) et/ou le dispositif de raccordement pivoté (4) en position pivotée.

12. Cassette de nettoyage ou d'entretien (1) selon la revendication 11, **caractérisée en ce que** l'élément d'enclenchement (21) est constitué par une saillie de la première et/ou seconde partie de boîtier (2, 3).

13. Cassette de nettoyage ou d'entretien (1) selon une des revendications précédentes, **caractérisée en ce que** le boîtier et le dispositif de raccordement (4) de la cassette de nettoyage ou d'entretien (1) a une conformation symétrique en rotation.

14. Cassette de nettoyage ou d'entretien (1) selon une des revendications précédentes, **caractérisée en ce que** la commande pivotante (7) de la cassette de nettoyage ou d'entretien (1) a une conformation symétrique en rotation.
